# FASCICULE DE BREVET EUROPEEN

(11) **EP 3 356 326 B1**
(45) Date de publication et mention de la délivrance du brevet: **07.04.2021**
(21) Numéro de dépôt: 16785251.6
(22) Date de dépôt: 29.09.2016
(51) Int. Cl.: C07C 319/06, C07C 321/04, C12P 11/00

(54) **PROCÉDÉ DE PRODUCTION DE MERCAPTANS PAR HYDROGÉNOLYSE ENZYMATIQUE DE DISULFURES À L'AIDE D'HYDROGÈNE**
VERFAHREN ZUR HERSTELLUNG VON MERCAPTANEN DURCH WASSERSTOFFUNTERSTÜTZTE DISULFID-ENZYMHYDROGENOLYSE
METHOD FOR PRODUCING MERCAPTANS BY HYDROGEN-ASSISTED DISULFIDE ENZYME HYDROGENOLYSIS

(30) Priorité: 30.09.2015 FR 1559264
(43) Date de publication de la demande: 08.08.2018
(73) Titulaire: Arkema France, 92700 Colombes (FR)
(72) Inventeur: FREMY, Georges, 64390 Sauveterre De Bearn (FR); MASSELIN, Arnaud, 35400 Saint Malo (FR); BRASSELET, Hugo, 44330 Vallet (FR)
(86) Numéro de dépôt international: PCT/FR2016/052480
(87) Numéro de publication internationale: WO 2017/055753

(56) Documents cités:
- US-A- 4 059 636
- US-A1- 2005 260 250
- SZAJEWSKI R P ET AL: "RATE CONSTANTS AND EQUILIBRIUM CONSTANTS FOR THIOL-DISULFIDE INTERCHANGE REACTIONS INVOLVING OXIDIZED GLUTATHIONE", JOURNAL OF THE AMERICAN CHEMICAL SOCIETY, AMERICAN CHEMICAL SOCIETY, US, vol. 102, no. 6, mars 1980 (1980-03), pages 2011-2025, XP002072983, ISSN: 0002-7863, DOI: 10.1021/JA00526A042
- MILLIS KEVIN K ET AL: "Oxidation/reduction potential of glutathione", THE JOURNAL OF ORGANIC CHEMISTRY, AMERICAN CHEMICAL SOCIETY, US, vol. 58, no. 15, janvier 1993 (1993-01), pages 4144-4146, XP009099411, ISSN: 0022-3263, DOI: 10.1021/JO00067A060
- MATTHEW J. G. STEWART ET AL: "Mycothiol disulfide reductase: solid phase synthesis and evaluation of alternative substrate analogues", ORGANIC & BIOMOLECULAR CHEMISTRY, vol. 6, no. 2, janvier 2008 (2008-01), page 385, XP055116173, ISSN: 1477-0520, DOI: 10.1039/b716380k
- DAVID A. KEIRE ET AL: "Kinetics and equilibria of thiol/disulfide interchange reactions of selected biological thiols and related molecules with oxidized glutathione", THE JOURNAL OF ORGANIC CHEMISTRY, vol. 57, no. 1, janvier 1992 (1992-01), pages 123-127, XP055257183, US ISSN: 0022-3263, DOI: 10.1021/jo00027a023
- MING DU ET AL: "Derivation of Oridonin with Bioreduction-Responsive Disulfide Bond", CHINESE JOURNAL OF CHEMISTRY, vol. 32, no. 5, 22 mai 2014 (2014-05-22), pages 448-453, XP055257871, CN ISSN: 1001-604X, DOI: 10.1002/cjoc.201300761
- SHUANG LIU ET AL: "Oligomeric Hydrogels Self-Assembled from Reduction-Controlled Condensation", ANGEWANDTE CHEMIE INTERNATIONAL EDITION, vol. 54, no. 12, 16 mars 2015 (2015-03-16) , pages 3639-3642, XP055257879, DE ISSN: 1433-7851, DOI: 10.1002/anie.201409952

## Description

La présente invention concerne un procédé de production par catalyse enzymatique de mercaptans, en particulier de méthylmercaptan, à partir de disulfures, en particulier de disulfure de diméthyle, et à l'aide d'hydrogène.

Les mercaptans sont d'une grande utilité dans de très nombreux domaines, par exemple comme arômes, odorisants pour gaz, agents de transfert de chaîne en polymérisation, matières premières pour l'industrie pharmaceutique ou cosmétique, pour la synthèse d'antioxydants, d'additifs extrême pression ou anti-usure pour la lubrification. Ces exemples ne sont en aucun cas limitatifs des utilisations des mercaptans connus aujourd'hui et qui peuvent être préparés grâce au procédé de l'invention.

En particulier, le premier des mercaptans, le méthylmercaptan (CH₃SH), présente un grand intérêt industriel, en particulier comme matière première de la synthèse de méthionine, acide aminé essentiel très largement utilisé dans l'alimentation animale. Le méthylmercaptan est également une matière première très largement utilisée pour la synthèse de nombreuses autres molécules.

Les mercaptans peuvent être synthétisés par de nombreuses méthodes telles que la sulfhydratation d'alcools, l'addition catalytique ou photochimique d'hydrogène sulfuré sur des composés organiques insaturés, la substitution à l'aide d'hydrogène sulfuré d'halogénures, d'époxydes ou de carbonates organiques, et autres.

En particulier, le méthylmercaptan est couramment produit en gros tonnage industriellement à partir de méthanol et de sulfure d'hydrogène selon la réaction (1) :

CH₃OH + H₂S → CH₃SH + H₂O (1)

Ces procédés présentent les inconvénients de nécessiter du méthanol (CH₃OH), de synthétiser l'hydrogène sulfuré (H₂S, à partir d'hydrogène et de soufre par exemple, d'où la nécessité de synthétiser de l'hydrogène également) et conduisent à des sous-produits de type éther diméthylique (CH₃OCH₃), sulfure de diméthyle (CH₃SCH₃) et produits de craquages et de l'eau, ce qui sous-entend de nombreuses étapes de purification du méthylmercaptan.

On trouvera la description de procédés basés sur ces réactions, à titre d'exemples, dans les demandes de brevets telles que WO2013092129, WO2008118925, WO2007028708, WO2006015668, WO2004096760.

Il peut se révéler intéressant économiquement (pour éviter la synthèse du méthanol) de vouloir produire le méthylmercaptan à partir de monoxyde de carbone, d'hydrogène et de sulfure d'hydrogène selon le schéma de synthèse (2) suivant :

CO + 2 H₂ + H₂S → CH₃SH + H₂O (2)

Cependant, ces procédés présentent les inconvénients de nécessiter du gaz de synthèse (CO/H₂) et donc de procéder à un reformage à la vapeur d'une source hydrocarbonée, d'avoir les bonnes proportions entre CO et H₂ donc de pouvoir ajuster le ratio CO/H₂ avec la réaction dite du « gaz à l'eau » (CO + H₂O → CO₂ + H₂), et de synthétiser l'H₂S.

Ces procédés conduisent également généralement à de fortes proportions de CO₂ en tant que sous-produit, ainsi qu'à du méthane, du sulfure de diméthyle et de l'eau. On trouvera des descriptions de ces procédés à titre d'exemple dans les demandes de brevets telles que US2010286448, US2010094059, US2008293974, US2007213564.

D'autres procédés encore ont été décrits et combinent différentes réactions telles que :
- Formation de CS₂ et d'H₂S à partir de méthane et de soufre (3) :

   CH₄ + 4 S → CS₂ + 2 H₂S (3)
- Hydrogénation du CS₂ (4) :

   CS₂ + 3 H₂ → CH₃SH + H₂S (4)

Il est également possible d'utiliser l'H₂S excédentaire des réactions (3) et (4) dans les réactions avec du méthanol (réaction 1) ou du gaz de synthèse (réaction 2) pour donner à nouveau du méthylmercaptan.

Ces procédés combinent évidemment les inconvénients décrits pour les réactions (1) et (2) avec la difficulté supplémentaire d'avoir de l'hydrogène excédentaire pour effectuer la réaction (4). On trouvera des descriptions de ces procédés dans les demandes de brevets US2011015443 ou, plus spécifiquement sur la réaction (4), dans la demande WO2010046607.

La demande WO200196290 propose un procédé de synthèse de méthylmercaptan directement à partir de méthane et d'H₂S avec coproduction d'hydrogène. Cette réaction directe entre le méthane et l'H₂S se fait à l'aide d'un plasma pulsé avec décharge en couronne. Cette demande ne décrivant aucun exemple de synthèse, il peut paraître difficile d'imaginer un procédé de synthèse industriel de méthylmercaptan à grande échelle avec cette technologie. De plus ce procédé nécessite la synthèse de l'H₂S si celui-ci n'est pas disponible.

La demande de brevet EP0649837 propose quant à elle un procédé de synthèse de méthylmercaptan par hydrogénolyse catalytique, avec des sulfures de métaux de transition, du disulfure de diméthyle avec de l'hydrogène. Ce procédé, bien que performant, nécessite des températures relativement élevées de l'ordre de 200°C pour obtenir des productivités intéressantes industriellement.

L'homme de l'art sait également qu'il est possible de préparer du méthylmercaptan par acidification d'une solution aqueuse de méthylmercaptide de sodium (CH₃SNa). Cette méthode présente l'inconvénient majeur de produire de grandes quantités de sels, tels que le chlorure de sodium ou le sulfate de sodium, selon que l'on utilise l'acide chlorhydrique ou l'acide sulfurique. Ces solutions aqueuses salines sont souvent très difficiles à traiter et les traces de produits malodorants qui subsistent font que cette méthode est difficilement envisageable sur le plan industriel.

Les procédés de synthèse des mercaptans supérieurs au méthylmercaptan présentent également de nombreux inconvénients. Ainsi la substitution des alcools par l'hydrogène sulfuré nécessite des températures et souvent des pressions élevées, et conduit à des sous-produits non-désirés de type oléfines, éthers et sulfures.

L'addition catalytique ou photochimique de l'hydrogène sulfuré sur des composés insaturés se fait généralement dans des conditions légèrement plus douces que précédemment mais conduit également à de nombreux sous-produits formés par isomérisation de la matière première, par addition non-régiosélective ou par double addition qui donne des sulfures. Enfin, la substitution de dérivés halogénés conduit à des procédés qui génèrent beaucoup d'effluents et de rejets salins difficilement compatibles avec des procédés industriels.
Dans le domaine de la biologie, plusieurs articles décrivent l'utilisation du couple glutathion/disulfure de glutathion (ou système GSH/GSSG) dans des réactions d'oxydo-réductions. L'article de R. P. Szajewski, (1980, Thiol-Disulfide Interchange reactions, American Chemical Society, p.2011-2025) décrit des thiols utilisés en tant que réducteurs du glutathion et transformés en disulfures.K. K. Millis et al., (J. Org. Chem. 1993, 58, 4144-4146) traite du potentiel de demi-cellule du système rédox glutathion/disulfure de glutathion. Un mélange comprenant du NADPH/NADP⁺, du GSH/GSSG et de la glutathion réductase est réalisé. Ming Du et al., (Chin. J. Chem. 2014, 32, 448-453) décrit l'utilisation de glutathion pour former un analogue de l'oridonine en réduisant un pont disulfure, à l'intérieur de cellules tumorales.
Shuang Liu et al. (Angew. Chem. Int. Ed. 2015, 54, 3639-3642) décrit la formation d'hydrogels oligomériques qui s'auto-assemblent par réduction de ponts disulfures grâce au glutathion. US 2005/0260250 traite de compléments alimentaires, permettant d'obtenir des thiols biodisponibles une fois ingérés grâce à plusieurs cycles antioxydants transmembranaires comprenant le glutathion.

Enfin, M. J. G. Stewart (Org. Biomol. Chem., 2008, 6, 385-390) décrit la synthèse et l'évaluation d'analogues du mycothiol, qui sont oxydés sous leur forme disulfure pour être testés en tant que substrat de l'enzyme mycothiol disulfure réductase. La présente invention a pour objet de proposer un nouveau procédé de préparation de mercaptans, en particulier de méthylmercaptan, qui ne présente pas les inconvénients décrits dans les procédés connus de l'art antérieur et précédemment détaillés.

Plus particulièrement, la présente invention a comme premier objet le procédé de préparation d'un mercaptan de formule R-SH, comprenant au moins les étapes de :
a) préparation d'un mélange comprenant :
   1) un disulfure de formule R-S-S-R',
   2) une quantité catalytique d'acide aminé porteur d'un groupement thiol ou d'un peptide à groupement thiol,
   3) une quantité catalytique d'une enzyme catalysant la réduction du pont disulfure créé entre deux équivalents dudit acide aminé porteur d'un groupement thiol ou dudit peptide à groupement thiol,
   4) une quantité catalytique d'une enzyme catalysant la réduction de l'hydrogène,
   5) une quantité catalytique d'un cofacteur commun aux deux enzymes catalysant la réduction et la déshydrogénation,
b) ajout d'hydrogène,
c) conduite de la réaction enzymatique,
d) récupération du mercaptan de formule R-SH et du mercaptan de formule R'-SH,
e) séparation et purification éventuelle du mercaptan de formule R-SH et/ou du mercaptan de formule R'-SH ; dans lequel
R et R', identiques ou différents, représentent indépendamment l'un de l'autre, un radical hydrocarboné, linéaire, ramifié ou cyclique, comportant de 1 à 20 atomes de carbone, ladite chaîne étant saturée ou porteuse d'une ou plusieurs insaturations sous forme de double(s) ou triple(s) liaison(s), R et R' pouvant également former ensemble et avec les atomes de soufre qui les portent une molécule cyclique comportant de 4 à 22 atomes, de préférence de 5 à 10 atomes.

L'enzyme catalysant la réduction de l'hydrogène peut être de tout type connu de l'homme du métier et par exemple l'enzyme hydrogène déshydrogénase.

D'une manière générale, l'enzyme catalysant la réduction du pont disulfure créé entre deux équivalents dudit acide aminé porteur d'un groupement thiol ou dudit peptide à groupement thiol est une enzyme réductase. Le terme « réductase » est utilisé dans la suite de la description pour l'explication de la présente invention.

Parmi les cofacteurs communs aux deux enzymes catalysant la réduction et la déshydrogénation (la réductase et la déshydrogénase), on peut citer à titre d'exemples non limitatifs les cofacteurs flaviniques, et les cofacteurs nicotiniques. On préfère utiliser les cofacteurs nicotiniques et plus particulièrement la Nicotinamide Adénine Dinucléotide (NAD), ou mieux encore la Nicotinamide Adénine Dinucléotide Phosphate (NADPH). Les cofacteurs listés ci-dessus sont avantageusement utilisés sous leurs formes réduites (par exemple NADPH, H+) et/ou leurs formes oxydées (par exemple NADP+), c'est-à-dire qu'ils peuvent être ajoutés sous ces formes réduites et/ou oxydées dans le milieu réactionnel.

Dans un mode de réalisation de l'invention, l'acide aminé porteur d'un groupement thiol et/ou le peptide porteur d'un groupement thiol peut être sous la forme du disulfure dudit acide aminé et/ou dudit peptide respectivement (par exemple glutathion sous forme de disulfure de glutathion).

L'organisation et l'ordre des ajouts des différents composants des étapes a) et b) du précédé défini ci-dessus peuvent être réalisés de différentes manières. Dans tous les cas, la réaction enzymatique de l'étape c) est déclenchée par ajout d'un des composants du système catalytique : soit une enzyme, soit un des composés ajoutés en quantité stœchiométrique (disulfure ou hydrogène) soit un des composés ajoutés en quantité catalytique (acide aminé porteur d'un groupement thiol ou peptide à groupement thiol ou du disulfure correspondant auxdites molécules ou bien encore le cofacteur).

Plus particulièrement encore, la présente invention a comme objet le procédé de préparation d'un mercaptan de formule R-SH, comprenant au moins les étapes de :
a') préparation d'un mélange comprenant :
   - un disulfure de formule R-S-S-R',
   - une quantité catalytique d'acide aminé porteur d'un groupement thiol ou d'un peptide à groupement thiol,
   - une quantité catalytique d'enzyme réductase correspondant audit acide aminé porteur d'un groupement thiol ou audit peptide à groupement thiol,
   - une quantité catalytique de NADPH,
b') ajout d'hydrogène avec une quantité catalytique d'enzyme hydrogène déshydrogénase,
c') conduite de la réaction enzymatique,
d') récupération du mercaptan de formule R-SH et du mercaptan de formule R'-SH,
e') séparation et purification éventuelle du mercaptan de formule R-SH et du mercaptan de formule R'-SH.

.

Selon un aspect préféré, les radicaux R et R', identiques ou différents, sont choisis indépendamment l'un de l'autre, parmi les radicaux alkyle, cycloalkyle, aryle, alkylaryle, arylalkyle, comportant de 1 à 20 atomes de carbone, de préférence de 1 à 12 atomes de carbone de préférence encore de 1 à 6 atomes de carbone, linéaires ou ramifiés, saturés ou non, et éventuellement fonctionnalisés par une ou plusieurs fonctions choisies, de manière non limitative et à titre d'exemples, parmi les fonction alcool, aldéhyde, cétone, acide, amide, nitrile, ester ou encore les fonctions porteuses de soufre, phosphore, silicium ou halogène.

Le disulfure de formule R-S-S-R' est susceptible d'être réduit, selon le procédé de l'invention, en mercaptan de formule R-SH et en mercaptan de formule R'-SH. Lorsque R est différent de R', on parle de disulfure dissymétrique, et lorsque R et R' sont identiques, on parle de disulfure symétrique. Dans le cas de disulfures symétriques R-S-S-R, le procédé de l'invention conduit à un mercaptan de formule R-SH. Selon un aspect tout particulièrement préféré de l'invention, on met en œuvre du disulfure de diméthyle (DMDS) dans le but de produire du méthylmercaptan CH₃SH.

Dans le cas de disulfures dissymétriques R-S-S-R', le procédé de l'invention conduit à un mélange des mercaptans de formules R-SH et R'-SH qui peut être soit utilisé tel quel ou bien soumis à une ou plusieurs opérations de séparation bien connues de l'homme du métier, par exemple la distillation.

Il est également possible de mettre en œuvre dans le procédé de l'invention des mélanges de un ou plusieurs disulfures, symétriques et/ou dissymétriques. Des mélanges de disulfures possibles peuvent comprendre les DSO (« DiSulfide Oils » en langue anglaise), lesdits DSO trouvant ainsi une possibilité de valorisation tout à fait intéressante.

Selon le procédé de l'invention, le ou les mercaptans produits sont généralement récupérés sous la forme d'un solide, d'un liquide et/ou d'un gaz.

Le procédé de production selon l'invention est basé sur la réduction enzymatique des disulfures, en particulier du disulfure de diméthyle, avec de l'hydrogène, selon la réaction suivante, illustrée avec le disulfure de diméthyle conduisant au méthylmercaptan :

CH₃SSCH₃ + H₂ → 2 CH₃SH

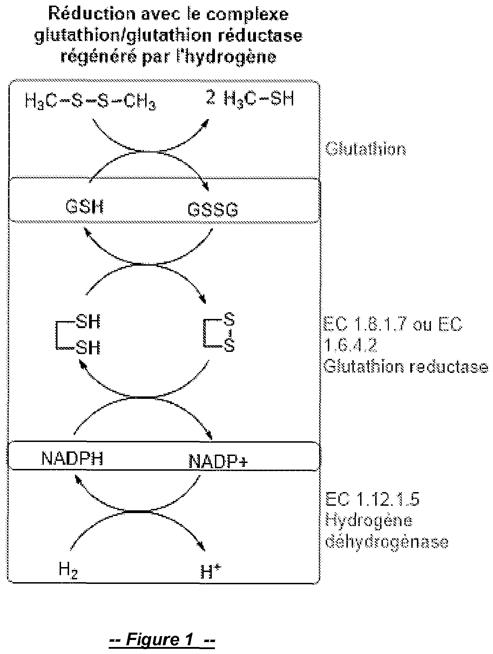

Il a maintenant été découvert que cette réaction est facilement catalysée par le système enzymatique mettant en œuvre un acide aminé à groupement thiol ou un peptide à groupement thiol, par exemple le glutathion, sous forme de complexe (acide aminé ou peptide)/enzyme réductase correspondante, régénéré par l'hydrogène, comme décrit à la Figure 1 annexée.

Ainsi selon l'illustration de la Figure 1, le peptide (exemple représenté le « glutathion ») réduit le disulfure (« DMDS » représenté) en mercaptan (« méthylmercaptan » représenté) en se transformant en peptide à pont disulfure (« disulfure de glutathion » représenté). L'enzyme réductase (« glutathion réductase » représentée, EC 1.8.1.7 ou EC 1.6.4.2) régénère le peptide (glutathion) et cette même enzyme est régénérée par un complexe enzymatique oxydo-réducteur bien connu de l'homme du métier, par exemple le complexe NADPH/NADP+ (Nicotine Adénine Dinucléotide Phosphate (forme réduite et forme oxydée)). À son tour NADP+ est régénéré en NADPH par l'intermédiaire de l'enzyme « Hydrogène déshydrogénase » (EC 1.12.1.5) grâce à de l'hydrogène. Le proton libéré par l'hydrogène ne s'accumule pas car il réagit avec la glutathion-réductase qui a donné HS-R-S⁻ après réaction avec NADPH et la fonction mercaptide formée devient une fonction mercaptan.

En d'autres termes, le peptide (« glutathion » représenté) réduit le disulfure (« DMDS » représenté) en mercaptan (« méthylmercaptan » représenté) en se transformant en peptide à pont disulfure (« disulfure de glutathion » représenté). L'enzyme catalysant la réduction (« glutathion réductase » représentée, avec les exemples de numéros de classification enzymatique EC 1.8.1.7 ou EC 1.6.4.2) régénère le peptide (« glutathion ») tout en oxydant le cofacteur (« NADPH,H+ » représenté). La forme oxydée (« NADP+ » représenté) est alors réduite à l'aide d'un complexe enzymatique oxydo-réducteur, dit « de recyclage », bien connu de l'homme de métier et comprenant l'enzyme déshydrogénase impliquée (« hydrogène déshydrogénase » représentée, avec l'exemple de numéro de classification enzymatique EC 1.1.1.47) et l'hydrogène. Le proton libéré par l'hydrogène ne s'accumule par car il réagit directement sur la fonction mercaptide formée pendant la réaction catalysée par l'enzyme réductase utilisée.

Selon un mode de réalisation tout particulièrement adapté, le système glutathion/disulfure de glutathion associé à l'enzyme glutathion-réductase permet selon la présente invention de réduire le DMDS en méthylmercaptan.

Le glutathion est un tripeptide largement utilisé en biologie. Cette espèce sous forme réduite (glutathion) ou oxydée (disulfure de glutathion) forme un couple d'oxydoréduction important dans les cellules. Ainsi le glutathion est vital pour supprimer les métaux lourds des organismes. Par exemple, la demande WO05107723 décrit une formulation où le glutathion est utilisé pour former une préparation chélatante, le brevet US4657856 enseigne que le glutathion permet également de détruire les peroxydes comme l'H₂O₂ en H₂O via la glutathion peroxydase. Enfin le glutathion permet également de réduire les ponts disulfures présents dans les protéines (Rona Chandrawati, « Triggered Cargo Release by Encapsulated Enzymatic Catalysis in Capsosomes », Nano Lett., (2011), vol. 11, 4958-4963).

Selon le procédé de l'invention, une quantité catalytique d'acide aminé porteur d'un groupement thiol ou d'un peptide à groupement thiol, est mise en œuvre pour la production de mercaptans à partir de disulfures.

Parmi les acides aminés porteurs de groupement thiol utilisables dans le procédé de la présente invention, on peut citer, à titre d'exemples non limitatifs la cystéine et l'homocystéine. Les systèmes enzymatiques oxydo-réducteurs utilisés régénérer le cycle catalytique de la même façon, sont dans ces cas le système cystéine/cystine-réductase EC 1.8.1.6, et homocystéine/homocystéine-réductase.

Parmi les peptides porteurs de groupement thiol utilisables dans le procédé de la présente invention, on peut citer, à titre d'exemples non limitatifs le glutathion et la thiorédoxine. Le système glutathion/glutathion réductase, décrit précédemment, peut ainsi être remplacé par le système thiorédoxine (CAS no. 52500-60-4)/thioredoxine réductase (EC 1.8.1.9 ou EC 1.6.4.5).

Le glutathion et le système glutathion/glutathion réductase sont tout particulièrement préférés pour la présente invention, en raison de la facilité d'approvisionnement et des coûts de ces composés.

Dans le procédé selon l'invention, l'hydrogène peut être ajouté au milieu réactionnel selon tout moyen connu de l'homme du métier, par exemple par bullage dans le milieu réactionnel qui est avantageusement un milieu réactionnel hydro-organique. La pression d'hydrogène dans le réacteur correspond à la pression du milieu réactionnel lui-même qui est définie plus loin.

L'enzyme utilisée est l'enzyme hydrogène-déshydrogénase, également bien connue de l'homme du métier.

Dans le procédé selon l'invention seuls les disulfure(s) et l'hydrogène sont utilisés en quantité stœchiométrique, tous les autres composants (acide aminé ou peptide, cofacteur (par exemple NADPH) et les 2 enzymes) sont utilisés en quantité catalytique.

Les avantages que procure le procédé de l'invention sont nombreux. Parmi ces avantages, on peut citer la possibilité de travailler en solution aqueuse ou hydro-organique, dans des conditions très douces de température et de pression et dans des conditions de pH proches de la neutralité. Toutes ces conditions sont typiques d'un procédé biocatalytique dit « vert» ou « durable ».

Un autre avantage lorsque le procédé met en œuvre du disulfure de diméthyle est que le méthylmercaptan produit, qui est à l'état gazeux dans les conditions de réaction, sort du milieu réactionnel au fur et à mesure de sa formation, accompagné éventuellement d'hydrogène qui n'aurait pas réagi. Le méthylmercaptan peut donc être directement utilisé à la sortie du réacteur dans une application plus en aval si l'hydrogène non réagi ne gêne pas dans cette dernière. Dans le cas contraire l'homme du métier saura aisément séparer l'hydrogène non converti du méthylmercaptan. Il peut aussi être facilement liquéfié par cryogénie par exemple si on souhaite l'isoler.

Le disulfure de diméthyle (DMDS) peut être produit sur un autre site à partir de méthylmercaptan et d'un oxydant tels que l'oxygène, le soufre ou l'eau oxygénée par exemple, ou encore à partir de sulfate de diméthyle et de disulfure de sodium. Le DMDS peut aussi provenir d'une source de « DiSulfide Oils » (DSO), comme indiqué précédemment, puis purifié par exemple par distillation réactive, comme décrit dans la demande WO2014033399. À noter que les DSO peuvent aussi être utilisés tels quels, sans nécessité de purification entre les différents disulfures les composant. On obtient alors un mélange de mercaptans en appliquant le procédé de l'invention.

Lorsque le DMDS est utilisé comme disulfure, le procédé selon l'invention est peut alors être considéré comme un procédé permettant d'éviter le transport de méthylmercaptan de son site de production par les voies existantes industrielles, vers son site d'utilisation, s'ils sont différents. En effet, le méthylmercaptan est un gaz à température ambiante, toxique et fortement malodorant ce qui complique énormément son transport déjà très réglementé contrairement au DMDS. Le procédé décrit dans la présente invention peut donc être utilisé pour produire du méthylmercaptan directement sur le site d'utilisation de ce dernier.

Le DMDS étant consommé dans la réaction et le méthylmercaptan sortant du milieu réactionnel au fur et à mesure de sa formation, sans hydrogène, ou avec de l'hydrogène non converti, aucun produit ne s'accumule dans le milieu réactionnel, dans l'hypothèse d'une alimentation en continu d'hydrogène et de DMDS. Il n'est donc pas nécessaire de recycler le système catalytique, au regard des produits qui entrent et qui sortent du réacteur.

Dans le cas d'autres disulfures, en fonction du point d'ébullition du mercaptan formé et de sa solubilité dans le milieu réactionnel, le mercaptan peut éventuellement décanter du milieu réactionnel pour être aisément séparé selon des techniques bien connues de l'homme du métier. Dans le cas contraire il pourra être isolé du milieu réactionnel, également par tout moyen connu de l'homme du métier.

De manière générale, la température de la réaction est comprise dans un domaine allant de 10°C à 50°C, de préférence entre 15°C et 45°C, de préférence encore entre 20°C et 40°C.

Le pH de la réaction peut être compris entre 6 et 8,5, de préférence entre 7,0 et 8,0. Le pH du milieu réactionnel peut être ajusté au moyen d'un tampon. De façon tout à fait préférée, on choisira le pH d'un milieu tamponné à une valeur comprise entre 7,5 et 8,0.

La pression utilisée pour la réaction peut aller d'une pression réduite par rapport à la pression atmosphérique à plusieurs bars (plusieurs centaines de kPa), selon les réactifs utilisés et le matériel utilisé. De façon préférée, on utilisera une pression allant de la pression atmosphérique à 20 bars (2 MPa) et de façon encore plus préférée on travaillera sous une pression allant de la pression atmosphérique à 3 bars (300 kPa).

Le procédé selon l'invention peut être réalisé en batch ou en continu, dans un réacteur en verre ou en métal suivant les conditions opératoires retenues et les réactifs utilisés. De façon préférée on choisit un procédé semi-continu dans lequel l'hydrogène est ajouté au fur et à mesure de sa consommation dans la réaction.

Le ratio molaire hydrogène/disulfure idéal est la stœchiométrie (ratio molaire = 1) mais peut varier de 0,01 à 100 si l'homme du métier y trouve un intérêt quelconque, telle qu'une addition en continu de l'hydrogène, alors que le disulfure est introduit dès le départ dans le réacteur. De façon préférée ce ratio molaire est choisi entre 1 et 20 globalement sur l'ensemble de la réaction.

L'hydrogène qui ne serait pas converti peut être recyclé de la sortie du réacteur à l'entrée du réacteur, jusqu'à épuisement total. On peut également considérer une boucle avec l'hydrogène et le(s) mercaptan(s) formé(s), jusqu'à ce que l'hydrogène ait complètement converti le(s) disulfure(s). En résultat, en fin de réaction lorsque la totalité de disulfure de diméthyle est converti, les gaz de sortie contiennent quasiment exclusivement du méthylmercaptan.

Les éléments présents en quantité catalytique dans le mélange préparé à l'étape a) ci-dessus (acide aminé porteur d'un groupement thiol ou d'un peptide à groupement thiol, enzyme réductase, cofacteur (par exemple NADPH)) sont aisément accessibles dans le commerce ou peuvent être préparés selon des techniques bien connues de l'homme du métier. Ces différents éléments peuvent se présenter sous forme solide ou liquide et peuvent très avantageusement être mis en solution dans l'eau pour être mis en œuvre dans le procédé de l'invention. Les enzymes utilisées peuvent également être greffées sur support (cas des enzymes supportées).

La solution aqueuse de complexe enzymatique comprenant l'acide aminé ou le peptide peut également être reconstituée par les méthodes connues par l'homme de métier, par exemple par perméabilisation de cellules qui contiendraient ces éléments. Cette solution aqueuse dont une composition est donnée dans l'exemple 1 suivant peut être utilisée dans des teneurs en masse comprises entre 0,01% et 20% par rapport au poids total du milieu réactionnel. De façon préférée on utilisera une teneur comprise entre 0,5% et 10%.

Il est également décrit l'utilisation d'une solution aqueuse de complexe enzymatique comprenant un acide aminé porteur d'une fonction thiol tel que défini précédemment ou d'un peptide porteur d'une fonction thiol tel que défini précédemment, pour la synthèse d'un mercaptan à partir d'un disulfure.

Le mélange pouvant être utilisé pour l'étape a) du procédé décrit précédemment et comprenant :
1) un disulfure de formule R-S-S-R',
2) une quantité catalytique d'acide aminé porteur d'un groupement thiol ou d'un peptide à groupement thiol,
3) une quantité catalytique d'une enzyme catalysant la réduction du pont disulfure créé entre deux équivalents dudit acide aminé porteur d'un groupement thiol ou dudit peptide à groupement thiol,
4) une quantité catalytique d'une enzyme catalysant la réduction de l'hydrogène,
5) une quantité catalytique d'un cofacteur commun aux deux enzymes catalysant la réduction et la déshydrogénation,
6) et éventuellement de l'hydrogène,
où R et R' sont tels que définis précédemment,
est nouveau et à ce titre fait partie de la présente invention.

Dans un mode de réalisation de l'invention, l'acide aminé porteur d'un groupement thiol et/ou le peptide porteur d'un groupement thiol peut être sous la forme du disulfure dudit acide aminé et/ou dudit peptide respectivement. Selon encore un mode de réalisation, le cofacteur est le NADPH sous sa forme oxydée (NADP+) ou sous sa forme réduite (NADPH, H+).

Plus particulièrement, ledit mélange comprend :
- un disulfure de formule R-S-S-R',
- une quantité catalytique d'acide aminé porteur d'un groupement thiol ou d'un peptide à groupement thiol,
- une quantité catalytique d'enzyme réductase correspondant audit acide aminé porteur d'un groupement thiol ou audit peptide à groupement thiol, et
- une quantité catalytique de NADPH,
où R et R' sont tels que définis précédemment.

On comprendra mieux l'invention avec les exemples suivants non limitatifs par rapport à la portée de l'invention. Tous les essais présentés ci-dessous ont été réalisés dans des conditions anaérobies.

### EXEMPLE 1 :

Dans un réacteur contenant 150 mL de solution aqueuse tamponnée à pH 7,8, sont introduits 10 mL de complexe enzymatique au glutathion. La solution de complexe enzymatique contient: 185 mg (0,6 mmol) de glutathion, 200 U de glutathion-réductase, 50 mg (0,06 mmol) de NADPH et 200 U d'enzyme hydrogène-déshydrogénase. Le milieu réactionnel est porté à 35°C sous agitation mécanique. Un premier prélèvement est effectué t = 0. Par la suite, le disulfure de diméthyle (9,4 g, 0,1 mol) est placé dans une burette et ajouté au goutte à goutte dans le réacteur. Dans le même temps, un flux d'hydrogène de 4 L.h⁻¹ (mesurés dans les conditions normales de température et de pression) est introduit par bullage dans le réacteur. La réaction est effectuée à la pression atmosphérique. Une analyse en chromatographie en phase gazeuse des gaz sortant du réacteur montre quasi essentiellement la présence d'hydrogène et de méthylmercaptan (quelques traces d'eau). Ces gaz de sortie sont piégés dans de l'hydroxyde de sodium (soude) à 20% dans l'eau. Le DMDS et l'hydrogène (ratio molaire hydrogène/DMDS sur l'ensemble de la réaction = 10,7) sont introduits en 6 heures et la réaction est suivie par dosage potentiométrique en argentimétrie du sel de sodium du méthylmercaptan, dans le piège en sortie de réacteur. L'analyse finale montre que le DMDS a été converti de façon quantitative en méthylmercaptan. De plus, une analyse finale en chromatographie en phase gazeuse du milieu réactionnel confirme l'absence de DMDS, et de méthylmercaptan qui a été chassé du réacteur par l'hydrogène en excès.

### EXEMPLE 2 :

Au milieu réactionnel de l'exemple 1, on réintroduit 9,4 g (0,1 mol) de DMDS au goutte à goutte en 6 heures, mais cette fois on n'introduit qu'un débit de 1 L.h⁻¹ d'hydrogène pendant 6 heures également (ratio molaire hydrogène/DMDS sur l'ensemble de la réaction = 2,7). Le suivi de la réaction se fait de la même façon que dans l'exemple 1 après avoir changé la solution de soude à 20% en sortie du réacteur. Les analyses en fin de réaction confirment la disparition complète du DMDS totalement converti en méthylmercaptan retrouvé sous forme de sel de sodium dans la solution de soude. Cet exemple montre la robustesse du système catalytique par sa reproductibilité et montre également que l'on peut travailler avec des ratios molaires hydrogène/DMDS qui se rapprochent de la stœchiométrie.

### EXEMPLE 3

Dans un réacteur contenant 70 mL de solution aqueuse tamponnée à pH 6,8, sont introduits 10 mL du complexe enzymatique au glutathion. La solution de complexe enzymatique contient : 200 mg (0,65 mmol) de glutathion, 500 U de glutathion-réductase, 100 mg (0,12 mmol) de NADPH et 50 U d'hydrogène déshydrogénase. Cette dernière est obtenue à partir de culture de microorganismes (selon Biller et collaborateurs, « Fermentation Hyperthermophiler Mikroorganismen am Beispiel von Pyrococcus Furiosus », Shaker Verlag, Maastricht/Herzogenrath, 2002) à l'aide de techniques bien connues de l'homme de métier.

Le milieu réactionnel est porté à 35°C sous agitation mécanique et balayage d'azote. Un premier prélèvement est effectué à t = 0. Par la suite, 20 g (0,22 mol) de disulfure de diméthyle sont ajoutés à l'aide d'une seringue.

Dans le même temps, une quantité de 4 L.h⁻¹ d'hydrogène (mesurés dans les conditions normales de température et de pression) est introduite par bullage dans le milieu réactionnel. La réaction est effectuée à pression atmosphérique.

Une analyse en chromatographie en phase gazeuse des gaz sortant du réacteur montre quasi essentiellement la présence d'hydrogène, d'azote et de méthylmercaptan (quelques traces d'eau). Ces gaz de sortie sont piégés dans l'hydroxyde de sodium à 20% en poids dans l'eau. Le DMDS et l'hydrogène (ratio molaire hydrogène/DMDS sur l'ensemble de la réaction = 4,9) sont introduits en 6 heures et la réaction est suivie par dosage potentiométrique en argentimétrie du sel de sodium de méthylmercaptan, dans le piège de sortie de réacteur.

L'analyse finale montre que le DMDS a été converti de façon quantitative en méthylmercaptan. De plus, une analyse finale en chromatographie en phase gazeuse du milieu réactionnel confirme l'absence de DMDS et de méthylmercaptan qui a été chassé du réacteur par l'hydrogène.

## Revendications

1. Procédé de préparation d'un mercaptan de formule R-SH, comprenant au moins les étapes de :
a) préparation d'un mélange comprenant :
1) un disulfure de formule R-S-S-R',
2) une quantité catalytique d'acide aminé porteur d'un groupement thiol ou d'un peptide à groupement thiol,
3) une quantité catalytique d'une enzyme catalysant la réduction du pont disulfure créé entre deux équivalents dudit acide aminé porteur d'un groupement thiol ou dudit peptide à groupement thiol,
4) une quantité catalytique d'une enzyme catalysant la réduction de l'hydrogène,
5) une quantité catalytique d'un cofacteur commun aux deux enzymes catalysant la réduction et la déshydrogénation,
b) ajout d'hydrogène,
c) conduite de la réaction enzymatique,
d) récupération du mercaptan de formule R-SH et du mercaptan de formule R'-SH,
e) séparation éventuelle et purification éventuelle du mercaptan de formule R-SH et/ou du mercaptan de formule R'-SH ;
dans lequel R et R', identiques ou différents, représentent indépendamment l'un de l'autre un radical hydrocarboné, linéaire, ramifié ou cyclique, comportant de 1 à 20 atomes de carbone, ladite chaîne étant saturée ou porteuse d'une ou plusieurs insaturations sous forme de double(s) ou triple(s) liaison(s) ou
R et R' forment ensemble et avec les atomes de soufre qui les portent une molécule cyclique comportant de 4 à 22 atomes.

2. Procédé selon la revendication 1 comprenant au moins les étapes de :
a') préparation d'un mélange comprenant :
• un disulfure de formule R-S-S-R',
• une quantité catalytique d'acide aminé porteur d'un groupement thiol ou d'un peptide à groupement thiol,
• une quantité catalytique d'enzyme réductase correspondant audit acide aminé porteur d'un groupement thiol ou audit peptide à groupement thiol,
• une quantité catalytique de NADPH,
b') ajout d'hydrogène avec une quantité catalytique d'enzyme hydrogène déshydrogénase,
c') conduite de la réaction enzymatique,
d') récupération du mercaptan de formule R-SH et du mercaptan de formule R'-SH,
e') séparation et purification éventuelle du mercaptan de formule R-SH et du mercaptan de formule R'-SH.

3. Procédé selon l'une quelconque des revendications précédentes, dans lequel les radicaux R et R', identiques ou différents, sont choisis indépendamment l'un de l'autre, parmi les radicaux alkyle, cycloalkyle, aryle, alkylaryle, arylalkyle, comportant de 1 à 20 atomes de carbone, linéaires ou ramifiés, saturés ou non, et éventuellement fonctionnalisés par une ou plusieurs fonctions choisies parmi les fonction alcool, aldéhyde, cétone, acide, amide, nitrile, ester ou encore les fonctions porteuses de soufre, phosphore, silicium ou halogène.

4. Procédé selon l'une quelconque des revendications précédentes, dans lequel le disulfure de formule R-S-S-R' est le disulfure de diméthyle.

5. Procédé selon l'une quelconque des revendications précédentes, dans lequel l'acide aminé porteur de groupement thiol ou le peptide porteur de groupement thiol est choisi parmi la cystéine, l'homocystéine, le glutathion et la thiorédoxine.

6. Procédé selon l'une quelconque des revendications précédentes, dans lequel l'hydrogène est introduit par bullage dans le milieu réactionnel.

7. Procédé selon l'une quelconque des revendications précédentes, dans lequel le pH de la réaction est compris entre 6 et 8,5.

8. Procédé selon l'une quelconque des revendications précédentes, dans lequel le pH de la réaction est compris entre 7,0 et 8,0.

9. Procédé selon l'une quelconque des revendications précédentes, dans lequel le ratio molaire hydrogène/disulfure est compris entre 0,01 et 100 sur l'ensemble de la réaction.

10. Procédé selon l'une quelconque des revendications précédentes, dans lequel le ratio molaire hydrogène/disulfure est compris entre entre 1 et 20 sur l'ensemble de la réaction.

11. Mélange comprenant :
1) un disulfure de formule R-S-S-R',
2) une quantité catalytique d'acide aminé porteur d'un groupement thiol ou d'un peptide à groupement thiol,
3) une quantité catalytique d'une enzyme catalysant la réduction du pont disulfure créé entre deux équivalents dudit acide aminé porteur d'un groupement thiol ou dudit peptide à groupement thiol,
4) une quantité catalytique d'une enzyme catalysant la réduction de l'hydrogène,
5) une quantité catalytique d'un cofacteur commun aux deux enzymes catalysant la réduction et la déshydrogénation,
6) et éventuellement de l'hydrogène,
où R et R' sont tels que définis dans la revendication 1.

12. Mélange selon la revendication 11 comprenant :
• un disulfure de formule R-S-S-R',
• une quantité catalytique d'acide aminé porteur d'un groupement thiol ou d'un peptide à groupement thiol,
• une quantité catalytique d'enzyme réductase correspondant audit acide aminé porteur d'un groupement thiol ou audit peptide à groupement thiol, et
• une quantité catalytique de NADPH, et
• une quantité catalytique d'une enzyme deshydrogénase,
où R et R' sont tels que définis dans la revendication 1.

## Patentansprüche

1. Verfahren zur Herstellung eines Mercaptans der Formel R-SH, das mindestens die folgenden Schritte umfasst:
a) Herstellen einer Mischung, umfassend:
1) ein Disulfid der Formel R-S-S-R',
2) eine katalytisch wirksame Menge einer Aminosäure, die eine Thiolgruppe trägt, oder eines Peptids mit einer Thiolgruppe,
3) eine katalytisch wirksame Menge eines Enzyms, das die Reduktion der zwischen zwei Äquivalenten der Aminosäure, die eine Thiolgruppe trägt, oder des Peptids mit einer Thiolgruppe gebildeten Disulfidbrücke katalysiert,
4) eine katalytisch wirksame Menge eines Enzyms, das die Reduktion von Wasserstoff katalysiert,
5) eine katalytisch wirksame Menge eines Cofaktors, das den beiden Enzymen, die die Reduktion und die Dehydrierung katalysieren, gemein ist,
b) Zugabe von Wasserstoff,
c) Durchführen der enzymatischen Reaktion,
d) Gewinnen des Mercaptans der Formel R-SH und des Mercaptans der Formel R'-SH,
e) fakultative Abtrennung und fakultative Reinigung des Mercaptans der Formel R-SH und/oder des Mercaptans der Formel R'-SH;
wobei R und R', die gleich oder verschieden sind, unabhängig voneinander für einen linearen, verzweigten oder cyclischen Kohlenwasserstoffrest mit 1 bis 20 Kohlenstoffatomen stehen, wobei die Kette gesättigt ist oder eine oder mehrere Ungesättigtheiten in Form von Doppel- oder Dreifachbindungen trägt, oder
R und R' zusammen und mit den Schwefelatomen, an die sie gebunden sind, ein cyclisches Molekül mit 4 bis 22 Atomen bilden.

2. Verfahren nach Anspruch 1, das mindestens die folgenden Schritte umfasst:
a') Herstellen einer Mischung, umfassend:
• ein Disulfid der Formel R-S-S-R',
• eine katalytisch wirksame Menge einer Aminosäure, die eine Thiolgruppe trägt, oder eines Peptids mit einer Thiolgruppe,
• eine katalytisch wirksame Menge eines Reduktaseenzyms, das der Aminosäure, die eine Thiolgruppe trägt, oder dem Peptid mit einer Thiolgruppe entspricht,
• eine katalytisch wirksame Menge von NADPH,
b') Zugabe von Wasserstoff mit einer katalytisch wirksamen Menge eines Wasserstoffdehydrogenaseenzyms,
c') Durchführen der enzymatischen Reaktion,
d') Gewinnen des Mercaptans der Formel R-SH und des Mercaptans der Formel R'-SH,
e') Abtrennung und fakultative Reinigung des Mercaptans der Formel R-SH und des Mercaptans der Formel R'-SH.

3. Verfahren nach einem der vorhergehenden Ansprüche, wobei die Reste R und R', die gleich oder verschieden sind, unabhängig voneinander aus linearen oder verzweigten, gesättigten oder ungesättigten und gegebenenfalls durch eine oder mehrere Funktionen, die aus Alkohol-, Aldehyd-, Keton-, Säure-, Amid-, Nitril- oder Esterfunktionen oder auch Schwefel, Phosphor, Silicium oder Halogen tragenden Funktionen ausgewählt sind, funktionalisierten Alkyl-, Cycloalkyl-, Aryl-, Alkylaryl- und Arylalkylresten mit 1 bis 20 Kohlenstoffatomen ausgewählt sind.

4. Verfahren nach einem der vorhergehenden Ansprüche, wobei es sich bei dem Disulfid der Formel R-S-S-R' um Dimethyldisulfid handelt.

5. Verfahren nach einem der vorhergehenden Ansprüche, wobei die Aminosäure, die eine Thiolgruppe trägt, bzw. das Peptid, das eine Thiolgruppe trägt, aus Cystein, Homocystein, Glutathion und Thioredoxin ausgewählt wird.

6. Verfahren nach einem der vorhergehenden Ansprüche, wobei der Wasserstoff durch Durchperlen in das Reaktionsmedium eingetragen wird.

7. Verfahren nach einem der vorhergehenden Ansprüche, wobei der pH-Wert des Ansatzes zwischen 6 und 8,5 liegt.

8. Verfahren nach einem der vorhergehenden Ansprüche, wobei der pH-Wert des Ansatzes zwischen 7,0 und 8,0 liegt.

9. Verfahren nach einem der vorhergehenden Ansprüche, wobei das Wasserstoff/Disulfid-Molverhältnis über die gesamte Reaktion zwischen 0,01 und 100 liegt.

10. Verfahren nach einem der vorhergehenden Ansprüche, wobei das Wasserstoff/Disulfid-Molverhältnis über die gesamte Reaktion zwischen 1 und 20 liegt.

11. Mischung, umfassend:
1) ein Disulfid der Formel R-S-S-R',
2) eine katalytisch wirksame Menge einer Aminosäure, die eine Thiolgruppe trägt, oder eines Peptids mit einer Thiolgruppe,
3) eine katalytisch wirksame Menge eines Enzyms, das die Reduktion der zwischen zwei Äquivalenten der Aminosäure, die eine Thiolgruppe trägt, oder des Peptids mit einer Thiolgruppe gebildeten Disulfidbrücke katalysiert,
4) eine katalytisch wirksame Menge eines Enzyms, das die Reduktion von Wasserstoff katalysiert,
5) eine katalytisch wirksame Menge eines Cofaktors, das den beiden Enzymen, die die Reduktion und die Dehydrierung katalysieren, gemein ist,
6) und gegebenenfalls Wasserstoff,
wobei R und R' wie in Anspruch 1 definiert sind.

12. Mischung nach Anspruch 11, umfassend:
• ein Disulfid der Formel R-S-S-R',
• eine katalytisch wirksame Menge einer Aminosäure, die eine Thiolgruppe trägt, oder eines Peptids mit einer Thiolgruppe,
• eine katalytisch wirksame Menge eines Reduktaseenzyms, das der Aminosäure, die eine Thiolgruppe trägt, oder dem Peptid mit einer Thiolgruppe entspricht, und
• eine katalytisch wirksame Menge von NADPH und
• eine katalytisch wirksame Menge eines Wasserstoffdehydrogenaseenzyms,
wobei R und R' wie in Anspruch 1 definiert sind.

## Claims

1. Process for the preparation of a mercaptan of formula R-SH, comprising at least the steps of:
a) preparation of a mixture comprising:
1) a disulfide of formula R-S-S-R',
2) a catalytic amount of amino acid bearing a thiol group or of a thiol-group-containing peptide,
3) a catalytic amount of an enzyme catalyzing the reduction of the disulfide bridge created between two equivalents of said amino acid bearing a thiol group or of said thiol-group-containing peptide,
4) a catalytic amount of an enzyme catalyzing the reduction of hydrogen,
5) a catalytic amount of a cofactor common to the two enzymes catalyzing the reduction and the dehydrogenation,
b) addition of hydrogen,
c) carrying out of the enzymatic reaction,
d) recovery of the mercaptan of formula R-SH and the mercaptan of formula R'-SH,
e) optional separation and optional purification of the mercaptan of formula R-SH and/or of the mercaptan of formula R'-SH;
in which R and R', which are identical or different, represent independently of one another a linear, branched or cyclic hydrocarbon-based radical comprising from 1 to 20 carbon atoms, said chain being saturated or bearing one or more unsaturations in the form of double or triple bond(s) or
R and R' form together, and with the sulfur atoms bearing them, a cyclic molecule comprising from 4 to 22 atoms.

2. Process according to Claim 1, comprising at least the steps of:
a') preparation of a mixture comprising:
• a disulfide of formula R-S-S-R',
• a catalytic amount of amino acid bearing a thiol group or of a thiol-group-containing peptide,
• a catalytic amount of reductase enzyme corresponding to said amino acid bearing a thiol group or to said thiol-group-containing peptide,
• a catalytic amount of NADPH,
b') addition of hydrogen with a catalytic amount of hydrogen dehydrogenase enzyme,
c') carrying out of the enzymatic reaction,
d') recovery of the mercaptan of formula R-SH and of the mercaptan of formula R'-SH,
e') separation and optional purification of the mercaptan of formula R-SH and of the mercaptan of formula R'-SH.

3. Process according to either one of the preceding claims, in which the radicals R and R', which are identical or different, are chosen independently of one another from linear or branched, saturated or unsaturated alkyl, cycloalkyl, aryl, alkylaryl or arylalkyl radicals comprising from 1 to 20 carbon atoms and optionally functionalized by one or more functions chosen from alcohol, aldehyde, ketone, acid, amide, nitrile or ester functions or else functions bearing sulfur, phosphorus, silicon or halogen.

4. Process according to any one of the preceding claims, in which the disulfide of formula R-S-S-R' is dimethyl disulfide.

5. Process according to any one of the preceding claims, in which the amino acid bearing a thiol group or the peptide bearing a thiol group is chosen from cysteine, homocysteine, glutathione and thioredoxin.

6. Process according to any one of the preceding claims, in which the hydrogen is introduced into the reaction medium via bubbling.

7. Process according to any one of the preceding claims, in which the pH of the reaction is between 6 and 8.5.

8. Process according to any one of the preceding claims, in which the pH of the reaction is between 7.0 and 8.0.

9. Process according to any one of the preceding claims, in which the hydrogen/disulfide molar ratio is between 0.01 and 100 over the whole of the reaction.

10. Process according to any one of the preceding claims, in which the hydrogen/disulfide molar ratio is between 1 and 20 over the whole of the reaction.

11. Mixture comprising:
1) a disulfide of formula R-S-S-R',
2) a catalytic amount of amino acid bearing a thiol group or of a thiol-group-containing peptide,
3) a catalytic amount of an enzyme catalyzing the reduction of the disulfide bridge created between two equivalents of said amino acid bearing a thiol group or of said thiol-group-containing peptide,
4) a catalytic amount of an enzyme catalyzing the reduction of hydrogen,
5) a catalytic amount of a cofactor common to the two enzymes catalyzing the reduction and the dehydrogenation,
6) and optionally hydrogen,
where R and R' are as defined in Claim 1.

12. Mixture according to Claim 11 comprising:
• a disulfide of formula R-S-S-R',
• a catalytic amount of amino acid bearing a thiol group or a thiol-group-containing peptide,
• a catalytic amount of reductase enzyme corresponding to said amino acid bearing a thiol group or to said thiol-group-containing peptide, and
• a catalytic amount of NADPH, and
• a catalytic amount of a hydrogen dehydrogenase enzyme,
where R and R' are as defined in Claim 1.
